(19) Europäisches Patentamt
European Patent Office
Office européen des brevets

(11) **EP 2 401 960 A2**

(12) **EUROPÄISCHE PATENTANMELDUNG**

(43) Veröffentlichungstag:
**04.01.2012 Patentblatt 2012/01**

(51) Int Cl.:
*A61B 5/029* (2006.01)     *A61B 5/11* (2006.01)

(21) Anmeldenummer: **11004063.1**

(22) Anmeldetag: **17.05.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **17.05.2010 DE 102010020752**

(71) Anmelder: **Kreative Technologie LWU UG ( haftungsbeschränkt)**
**82279 Eching am Ammersee (DE)**

(72) Erfinder:
• **Tank, Volker, Dr.-Ing., Dr.-Ing. habil**
  **82279 Eching am Ammersee (DE)**
• **Tank, Johannes**
  **82279 Eching am Ammersee (DE)**

(54) **Verfahren und Vorrichtung zur Messung körperlicher Leistungsfähigkeit**

(57) Die Erfindung betrifft ein Verfahren und Vorrichtung zur Messung körperlicher Leistungsfähigkeit und insbesondere der Herzleistungsfähigkeit beim Ausdauertraining (Gehen, Laufen, Radfahren, Laufbandergometer, etc.). Dazu werden spezifische Bewegungsparameter eingeführt, die eine Quantifizierung der Leistungsfähigkeit in Abhängigkeit von den Trainingsparametern erlauben. Vier Messungen werden durchgeführt und verknüpft: Herzfrequenz, und Gewicht des Übenden, sowie Zeitdauer und Wegstrecke des Trainings. Die Leistungsfähigkeit sowie ihre Veränderungen in Abhängigkeit von Trainingsdauer und -intensität lassen sich ermitteln. Der Bezug auf die Herzfrequenz erlaubt die Bestimmung der durch das Training bewirkten Veränderungen der Herzleistung.

Fig. 1

EP 2 401 960 A2

**Beschreibung**

Die Erfindung betrifft ein Verfahren und eine Vorrichtung zur Bestimmung körperlicher Leistungsfähigkeit, insbesondere der Herzleistungsfähigkeit bei jeder Art von sportlicher Bewegung, mit einem Pulsmessgerät, einem Entfernungsmessgerät, einem Zeitmesser und unter Verwendung einer Waage, die mit einem Datenverarbeitungssystem verbunden sind, welches aus den Messdaten "Spezifische Leistungen", insbesondere eine "Spezifische Herzleistung", die alternativ auch "Herzleistung" genannt wird, bestimmt.

Zu- oder Abnahme der Spezifischen Herzleistung oder Herzleistung durch Training oder Trainingsmangel werden quantitativ ermittelt.

Die Erfindung kann bei jeder Bewegungsart und Ausdauersportart im Freien oder in Sportanlagen, sowie auch an Übungsgeräten in sog. Fitnesscentern verwendet werden.

Körperliche Aktivität ist in jedem Alter ein sicheres Mittel zur Erhaltung der Gesundheit, der Leistungsfähigkeit und der Lebensqualität. Fehlende körperliche Aktivität ist ein anerkannter Risikofaktor für Herz-Kreislauferkrankungen, die z. Zt. häufigste Todesursache in den Industrieländern. Die Wirkung körperlicher Aktivität sowohl zur Primärprävention als auch zur Rehabilitation kardiovaskulärer Erkrankungen ist vielfältig nachgewiesen. Regelmäßiges Ausdauertraining (Gehen, Laufen, Rad fahren, Schwimmen, u. ä. im freien Gelände, oder Übungen an Trainingsgeräten) von wöchentlich drei bis vier Einheiten je 30-45 Minuten führen zu ausreichenden Resultaten. Das Ausdauertraining bewirkt u. a. eine Kräftigung und Verbesserung der Durchblutung des Herzmuskels und vieler Körpermuskeln, eine Verbesserung der peripheren Durchblutung, des Fettstoffwechsels ("Fettverbrennung") u. ä.. Vor allem nimmt die Herzleistung zu, u. a. erkennbar an einer Verringerung des Ruhepulses und eines geringeren Pulses bei Belastung. Fehlendes Ausdauertraining führt zu einer Umkehr der Prozesse und Wirkungen. Die Trainingsintensität sollte 50-70 % der maximalen Leistungsfähigkeit betragen (Empfehlung der Deutschen Gesellschaft für Sportmedizin und Prävention DGSP). Als Maß für die Leistungsfähigkeit wird die Pulsfrequenz verwendet, die mit einem mobilen Herzfrequenzmessgerät (Pulsuhr) gemessen und vom Übenden überwacht wird. Handelsübliche Pulsuhren ermitteln und zeigen die Pulsfrequenz als Mittelwert aus Messungen über einige Sekunden Dauer an. Sie verfügen ferner meist über eine Uhr und eine Stoppuhrfunktion, sowie eine Anzeige der beim Gehen/Laufen verbrauchten Kalorien. Es sind auch technisch aufwendige und dann teure Pulsuhren erhältlich, die über ein Navigationssystem (z. B. GPS, Global Positioning System) verfügen, welches der Bestimmung der beim Training zurückgelegten Entfernung dient.

Ferner sind "Laufsensoren" verfügbar, die vorzugsweise am Fuß getragen werden und beim Gehen und Laufen in der Regel über Beschleunigungsmessungen die Geschwindigkeit und die Entfernung bestimmen.

Moderne Trainingsgeräte (Ergometer und Hometrainer, wie Crosstrainer, stationäre Fahrräder, u. ä.) sind häufig mit sog. "Trainingscomputern" ausgerüstet. Diese Geräte haben meist mehrere Anzeigefunktionen: Strecke, Zeit, Geschwindigkeit, Puls, Leistung, Energieumsetzung, Körperfettmessung, etc..

Mit Hilfe der genannten Messgeräte ist es möglich, Veränderungen der körperlichen Leistungsfähigkeit qualitativ festzustellen, aber weder die Trainingscomputer, noch die Pulsuhren, noch andere technische Hilfsmittel verfügen über Einrichtungen zur quantitativen Ermittlung der körperlichen Leistungsfähigkeit und insbesondere der Herzleistungsfähigkeit, bzw. Herzlesitung.

Der Übende, insbesondere mit Herz-Kreislauf-Risiko, der durch Ausdauertraining seine Gesundheit und Leistungsfähigkeit erhalten und gegebenenfalls steigern möchte, fände eine wertvolle Unterstützung in einem Messsystem, das ihm den jeweiligen Leistungsstand, z. B. in Form der Zu- oder Abnahme der Herzleistung, quantitativ anzeigt.

Unter Herzleistung versteht man in der Medizin das Schlagvolumen, die Blutfördermenge je Herzschlag. Die Herzleistung kann derzeit in medizinischen Einrichtungen mittels aufwendiger Geräte bestimmt werden, was in der Regel nur in medizinisch notwendigen Fällen durchgeführt wird.

Die Ermittlung der körperlichen Leistung beim Ausdauertraining könnte unter Anwendung einfacher physikalischer Gesetze aus der Ermittlung der geleisteten Arbeit erfolgen. Dazu müssten alle wirkenden Kräfte bekannt sein. Deren Messung ist technisch grundsätzlich möglich, indem Kraftsensoren in Form von Dehnungsmessstreifen, Piezokraftsensoren, u. ä. verwendet werden. Diese müssen am Ort der Kraftwirkung angebracht werden, beim Gehen z. B. an den Schuhsohlen. Für dauerhaften, häufigen Einsatz bei freier Bewegung wäre dazu ein großer Aufwand erforderlich. Sehr kompliziert wird es, wenn komplexe Bewegungen vorliegen (Schwimmen, Rudern, u. ä.) und wenn auch die Körperform eine Rolle spielt (z. B. beim Schwimmen). Es wird dann sehr schwierig, die wirkenden Kräfte - und daraus abgeleitet Leistungen - zu bestimmen. Es ist derzeit darüber hinaus nicht möglich, aus gemessenen körperlichen Leistungen die Herzleistung quantitativ abzuleiten.

Als Nachteil der existierenden Technik zur Unterstützung und Begleitung des Ausdauertrainings wird angesehen, dass mit ihr Leistungsmessungen in freier Bewegung nicht möglich sind und insbesondere keine quantitative Bestimmung der Herzleistung vorgenommen werden kann.

Nachteilig ist ferner, dass an Trainingsgeräten zwar Leistungsmessungen möglich sind, diese aber nur Aufschluss über die körperliche Leistungsfähigkeit geben, und dass auch hier keine quantitative Bestimmung der Herzleistung vorgenommen werden kann.

Insbesondere wird als nachteilig angesehen, dass Leistungsmesssysteme für Messungen bei freier Bewegung und für breite, kontinuierliche Anwendung nicht verfügbar sind. Dieses vor allem, weil gerade die Bewegung im Freien an der frischen Luft (Gehen, Laufen, Schwimmen, Radfahren, Skilanglauf, u. ä.) die empfohlene Trainingsform ist.

Als weiterer Nachteil wird angesehen, dass selbst unter Verwendung verfügbarer Messtechnik (Kraftsensoren) deren Dauereinsatz einen sehr großen Aufwand darstellen würde. Das liegt insbesondere daran, dass die Sensorik extremen Umweltbelastungen und damit hohem Verschleiß ausgesetzt wäre (z. B. zwischen Schuhsohle und Straßenbelag). Ferner ist die vorhandene Messtechnik in der Regel nicht dazu geeignet, von ungeschulten Laien richtig genutzt zu werden.

Aufgabe der Erfindung ist daher die Schaffung von Verfahren und Einrichtungen mit denen beim individuellen sportlichen Training unterschiedlicher Art in freier Bewegung und/oder an Übungsgeräten ständig und kontinuierlich der Stand der körperlichen Leistungsfähigkeit und insbesondere der Herzleistungsfähigkeit bestimmt werden können. Verfahren und Einrichtung sollen einfach zu verwenden sein, sodass sie ohne Vorkenntnisse sofort zum Einsatz kommen können. Ferner soll ihre Verwendung über lange Zeit (Jahre) ohne Verschleiß und wartungsfrei gewährt sein.

Zur Lösung der Aufgabe wird ein neues Verfahren zur quantitativen Bestimmung der Leistungsänderungen (Zu- oder Abnahme) des Übenden und seines Herzens im Verlauf des Ausdauertrainings und nach Trainingspausen entwickelt. Es wird dabei nicht die Volumenleistung des Herzens bestimmt, sondern eine neue bewegungsspezifische Herzleistung, die "Spezifische Herzleistung", oder auch "Herzleistung" genannt wird. Nachfolgend wird die Erfindung zunächst für die Anwendung bei allen Ausdauertrainingsarten beschrieben, die mit einer Fortbewegung (Zurücklegen einer Strecke) verknüpft sind (inklusive stationäre Laufbänder und stationäre Trainingsgeräte, bei denen eine Wegmessung durchgeführt wird oder werden kann und stationäre Trainingsgeräte bei denen eine Messung der im Training umgesetzten Energie durchgeführt wird oder werden kann). Vorrichtungen zur Durchführung des Verfahrens werden anschließend beschrieben.

Grundlage der Bestimmung der körperlichen und der Herzleistungsfähigkeit bei Bewegung ist die Ermittlung der geleisteten mechanischen Arbeit (Arbeit = Energie):

$$E = F * x * s \tag{1}$$

E: Arbeit
F: Kraft s: Weg

$$F = m * a \tag{2}$$

m: Masse
a: Beschleunigung

Aus (1) ergibt sich unter Berücksichtigung der für die Bewegung erforderlichen Zeit t die Leistung P:

$$P = E / t \tag{3}$$

P: Leistung
t: Zeit

Handelt es sich um reine Hubarbeit (z.B. beim Treppensteigen auf die Höhe h), dann ist die Masse m ist das Gesamtgewicht der Übenden (inkl. Kleidung) der Weg s ist die überwundene Höhe h und a ist die Gravitationsbeschleunigung g (g = 9,81 m/s$^2$). Bei anderen Bewegungsformen (Gehen, Laufen, Schwimmen, Radfahren, Rudern, Skilanglauf, u. ä.) ist die Bestimmung der geleisteten Arbeit schwieriger. Hier macht die Hubarbeit nur einen geringen oder gar keinen Teil der gesamten Arbeit aus, ein größerer Anteil besteht in der Arbeit zur Vorwärtsbewegung. Die zur Bestimmung der Arbeit notwendigen Messungen wären sehr aufwendig.

Erfindungsgemäß wird daher eine "Spezifische Trainingseinheit" definiert. Sie ist eine der möglichen Übungsarten (Gehen, Laufen, Rudern, etc., also "Spezifische Geheinheit", "Spezifische Laufeinheit", "Spezifische Rudereinheit", etc.), die wiederholt für eine Zeitdauer t über Trainingsperioden der Zeit T (die Zeit t kann dabei Sekunden, Minuten, Stunden betragen, die Zeit T kann Tage, Woche, Monate betragen) reproduzierbar durchgeführt wird. D. h. sie wird z.

B. auf immer der gleichen Strecke (gleiche Länge, gegebenenfalls gleiche Steigung, gleicher Untergrund, etc.) ausgeführt. Abhängig vom Trainingsziel wird in Verlauf der Trainingsperiode der Zeit T die Übungsintensität variiert, z. B. durch schnelleres/langsameres Gehen, Rudern, etc.. Die Spezifische Trainingseinheit (gleiche Strecke) wird also in kürzerer oder längerer Zeit t absolviert. Mehrere unterschiedliche Spezifische Trainingseinheiten können bei einer Ausdauerübung aufeinander folgen (z. B. Laufen in der Ebene, Gehen an einer Steigung, Radfahren, etc.).

Für jede einzelne der Spezifischen Trainingseinheiten wird basierend auf Gl. (1, 2) erfindungsgemäß als neue Größe die "Spezifische Bewegungsenergie" definiert. Ausgehend von:

$$E = F * s = m * a * s$$

wird die Beschleunigung a gleich konstant (z. B. zu Eins) gesetzt:

$$a = const$$

Die wirkenden Kräfte oder Beschleunigungen werden also nicht gemessen. Es ergibt sich:

$$E_B = m * const * s \qquad (5)$$

$E_B$: Spezifische Bewegungsenergie

Die Spezifische Bewegungsenergie ist je nach Training eine Spezifische Gehenergie, Spezifische Laufenergie, Spezifische Ruderenergie, etc. und hat die Einheit N bzw. kg m$^2$ / s$^2$.

Entsprechend (3) folgt daraus die "Spezifische Bewegungsleistung" (bzw. "Spezifische Übungsleistung"), als die in der Spezifischen Trainingseinheit erbrachte mittlere Leistung.

$$P_B = E_B / t \qquad (6)$$

t: Zeit, die für die Spezifische Trainingseinheit (Strecke) benötigt wird

$P_B$: Spezifische Bewegungsleistung

Die körperliche Leistung ist während einer Spezifischen Trainingseinheit nicht konstant, da einerseits der Übende seine Geschwindigkeit nicht exakt konstant halten kann und andererseits beim Üben im Gelände Steigungen und Gefälle, sowie Änderungen des Untergrunds, des Windes, etc. Leistungsänderungen zur Folge haben. Die Spezifische Bewegungsleistung ist daher der Mittelwert aller Momentanleistungswerte, die während der Spezifischen Trainingseinheit der Zeitdauer t auftreten. Die Zeitdauer t kann Sekunden (zur Erzielung hoher zeitlicher Auflösung), Minuten oder Stunden (z. B. beim Marathonlauf) betragen.

Auch hier wird je nach Trainingsart unterschieden zwischen Spezifischer Gehleistung, Spezifischer Laufleistung, Spezifischer Ruderleistung, etc., mit der Einheit W, bzw. kg m$^2$ / s$^3$.

Ferner wird erfindungsgemäß als neue Größe die "Spezifische Herzleistung" $P_H$ als Quotient aus Spezifischer Bewegungsleistung $P_B$ und Zahl $n_s$ der Herzschläge innerhalb der Zeit t nach Gl. (6) definiert:

$$P_H = P_B / n_S = (m * const * s) / (t * n_S) \qquad (7)$$

$P_H$: "Spezifische Herzleistung"

$n_s$: Zahl der Herzschläge

Die Spezifische Herzleistung (sie wird hier auch "Herzleistung" genannt) ist analog der Spezifischen Bewegungsleistung der Mittelwert aller Momentanleistungswerte, die während der Spezifischen Trainingseinheit der Zeitdauer t auftreten.

Wieder wird je nach Trainingsart unterschieden zwischen Spezifischer Geh-Herzleistung, Spezifischer Lauf-Herzleistung, Spezifischer Ruder-Herzleistung, etc., mit der Einheit W/S, bzw. (kg m$^2$) / (s$^3$ S), wobei "S" für Herzschlag steht.

Betrachtet man die Beschleunigung "const" in Gl. (7) als bedeutungslos und ignoriert sie (wodurch auch ihre Dimension verschwindet), dann bekommt die Spezifische Herzleistung die Einheit: (kg m) / (s S). Sie wird dadurch anschaulich, denn z. B. beim Gehen, Laufen oder Rudern, etc. gibt sie an, wie viele Kilogramm Meter pro Sekunde und Herzschlag geleistet (bewegt) werden.

In Erweiterung des Verfahrens wird im Training auch die Atemfrequenz gemessen und aufgezeichnet und eine "Spezifische Lungenleistung" bestimmt. In einer zusätzlichen Erweiterung werden auch Bewegungsparameter, wie Schrittzahl, Schlagzahl beim Rudern, etc. bestimmt und daraus eine "Spezifische Schrittleistung", eine "Spezifische Schlagleistung", etc. bestimmt. Aus allen aufgezeichneten Parametern wird eine "Spezifische Körperleistung" ermittelt.

Es wird eine "Spezifische Lungenleistung" definiert:

$$P_L = P_B \, / \, n_A \tag{8}$$

$P_L$: "Spezifische Lungenleistung"
$n_A$ : Zahl der Atemzüge

Diese Größe gibt Aufschluss über die Effizienz der Atmung, der Atemtechnik und des Gasaustausches über die Lunge. Sie wird u. a. beeinflusst von der Energieumsetzung im Organismus und der Herzleistungsfähigkeit (z. B. Kurzatmigkeit bei Herzinsuffizienz.). Auch hier wird je nach Trainingsart unterschieden, wie vorstehend beschrieben.

Es wird eine "Spezifische Schrittleistung" definiert:

$$P_S = P_B \, / \, n_{st} \tag{9}$$

$P_s$: "Spezifische Schrittleistung"
$n_{st}$: Zahl der Schritte

Diese Größe gibt Aufschluss über die Bewegungstechnik, Schrittweite, Leistungsfähigkeit der Beine (Muskeln) und wird beeinflusst vom Schuhwerk, Untergrund, u. ä.. Sie wird für andere Bewegungsabläufe (Schwimmen, Radfahren, Rudern, u. ä.) entsprechend modifiziert (also Tretkurbelumdrehungen, Ruderschläge anstelle Schrittzahl). Auch hier wird je nach Trainingsart unterschieden, wie vorstehend beschrieben. Gleichung (5) wird an die jeweilige Bewegungsart angepasst, z. B. beim Radfahren und Rudern wird die Gesamtmasse (Fahrrad mit Fahrer, Boot mit Insassen) ermittelt und verwendet.

Als umfassende Leistungsgröße wird unter Bezug der Spezifischen Bewegungsleistung auf die Zahl der Herzschläge, der Atemzüge und der Schrittzahl die "Spezifische Körperleistung" definiert:

$$P_K = P_B \, / \, (n_S * n_a * n_{st} \, ) \tag{10}$$

$P_K$: "Spezifische Körperleistung"

Aus G1. (7, 8, 9,10) ergeben sich unterschiedliche Werte für unterschiedliche Spezifische Trainingseinheiten (Gehen, Laufen, etc., bzw. ebener Weg, Steigung, etc.), die nicht direkt miteinander vergleichbar sind. Über eine Trainingsperiode der Zeit T (von Tagen, Wochen, Monaten) regelmäßig ermittelt, gibt jede aber denselben Einblick in die Veränderungen der Leistung. Dazu wird beispielsweise in einem Diagramm die jeweils gemessene Spezifische Leistung als Funktion des Zeitpunkts der jeweiligen Trainingseinheit dargestellt (Leistungs-Zeitdiagramm). Erfindungsgemäß werden die Spezifischen Leistungen bei unterschiedlicher Spezifischen Trainingseinheit direkt vergleichbar, indem jeweils ihre Änderungen als Funktion der Zeit als "Spezifische Trainingseffizienz η" bestimmt werden. Dazu wird die erste Ableitung der jeweiligen Spezifischen Leistung nach der Zeit T gebildet (nachfolgend für die Spezifische Bewegungsleistung und die Spezifische Herzleistung):

$$\eta_B = dP_B \, / \, dT \tag{11}$$

$\eta_B$: "pezifische Bewegungstrainingseffizienz"
T: Zeit in der Trainingsperiode (z. B. Datum)

$$\eta_H = dP_H \, / \, dT \tag{12}$$

$\eta_H$: "Spezifische Herztrainingseffizienz"

Ergibt sich $\eta_B$ bzw. $\eta_H$ positiv, dann hat die Leistung zugenommen (der Körper in der spezifischen Trainingsart, bzw. das Herz ist leistungsfähiger geworden), ist es negativ, dann hat die Leistung abgenommen (z. B. nach Trainingspausen), ist es Null, dann ist der Leistungsstand konstant. Die Größe von $\eta_B$ bzw. $\eta_H$ ist das Maß für die Zu- bzw. Abnahme der Körper-, bzw. Herzleistung. Die auch hier vorgenommene Unterscheidung nach Bewegungsart, in Form z. B. einer Spezifischen Gehtrainingseffizienz, oder Spezifischen Rudertrainingseffizienz, berücksichtigt, dass beispielsweise Gehtraining zwar die Gehleistungsfähigkeit steigert, nicht aber in gleichem Maße die Ruderleistungsfähigkeit, dass aber Gehtraining und Rudertraining in vergleichbarer Weise die Herzleistungsfähigkeit beeinflussen.

Auf diese Weise ist die Kombination verschiedener Bewegungsarten im Training möglich und vergleichbar.

Eine "Spezifische Lungentrainingseffizienz $n_L$" ergibt sich aus:

$$\eta_L = dP_L \, / \, dT \tag{13}$$

Eine "Spezifische Schritttrainingseffizienz $\eta_S$" ergibt sich aus:

$$\eta_S = dP_S \, / \, dT \tag{14}$$

Eine "Spezifische Körpertrainingseffizienz $\eta_K$" ergibt sich aus:

$$\eta_K = dP_K \, / \, dT \tag{15}$$

Alternativ werden die absoluten Änderungen der Spezifischen Bewegungsleistung $\Delta P_B(T)$, bzw. der Spezifischen Herzleistung $\Delta P_H(T)$, oder alternativ die prozentualen Änderungen $\Delta P_{Bp}(T)$, bzw. $\Delta P_{Hp}(T)$, in der Trainingsperiode der Zeit T berechnet. Dazu werden die Leistungen nach Gl. (6) und Gl. (7) auf einen Referenzwert bezogen. Der Referenzwert ist beispielsweise der Mittelwert $P_{Bm}$ bzw. $P_{Hm}$, oder alternativ der Anfangswert $P_{Ba}$, bzw. $P_{Ha}$ der Leistungen in der Trainingsperiode der Zeit T. Auch auf diese Weise werden erfindungsgemäß die Spezifischen Leistungen bei unterschiedlichen Trainingseinheiten und unterschiedlichen Trainingsarten direkt vergleichbar. Nachfolgend werden exemplarisch zwei der acht Möglichkeiten dargestellt:

$$\Delta P_B(T) = \{P_B(T) - P_{Bm}\} \, / \, P_{Bm} \tag{16}$$

$\Delta P_B(T)$: absolute Änderung der Spezifischen Bewegungsleistung, hier bezogen auf den Mittelwert

$P_{Bm}$: Mittelwert der Spezifischen Bewegungsleistung innerhalb der Trainingsperiode der Zeit T

$$\Delta P_{Hp}(T) = \{P_H(T) - P_{Ha}\} * 100 \, / \, P_{Ha} \tag{17}$$

$\Delta P_{Hp}(T)$: prozentuale Änderung der Spezifischen Herzleistung, hier bezogen auf den Anfangswert

$P_{Ha}$: Anfangswert der Spezifischen Herzleistung innerhalb der Trainingsperiode der Zeit T

Werden im Training unterschiedliche Spezifische Trainingseinheiten kombiniert, so werden für jede gesondert die Leistungen nach Gl. (6,7) ermittelt werden. Diese Leistungen sind unter den Teilstrecken nicht vergleichbar. Die nach Gl. (16) und Gl. (17) berechneten absoluten und prozentualen Leistungsänderungen sind unter den Teilstrecken vergleichbar und sind für eine Trainingsperiode der Zeit T innerhalb der Grenzen der Messunsicherheit gleich.

Vorzugsweise vor, alternativ auch während oder nach dem Training werden das Gewicht (Masse) der übenden Person und während des Trainings mittels mobiler Messsysteme Zeit, Entfernung und Puls gemessen und aufgezeichnet. Sie werden einem Datenauswerte-und -verwaltungssystem (das stationär ist oder mobil beim Training mitgeführt wird) übergeben, das daraus Leistungsparameter, insbesondere eine "Spezifische Bewegungsleistung" und eine "Spezifische Herzleistung" bestimmt, speichert und dem Übenden bei Bedarf zur Anzeige bringt. Das Datenverwaltungssystem speichert alle Trainingsdaten, sowie das jeweilige Datum, ermittelt die Trainingshistorie und zeigt sie in Form einer "Spezifischen Trainingseffizienz", bzw. einer absoluten oder einer prozentualen Leistungsänderung an.

Da die körperliche Leistung und die Herzleistung von einer Vielzahl von Einflüssen abhängen, sind sie auch bei konstantem Training und ausgewogener Lebensweise von Tag zu Tag leicht unterschiedlich ("Tagesform"). Gesundheitliche Beeinträchtigungen und außergewöhnliche Belastungen können die täglichen Schwankungen vergrößern. Sie repräsentieren daher nicht die tatsächlichen Zu- oder Abnahmen der Leistungsfähigkeit. Diese werden erst bei Betrachtung längerer Zeiträume (einige Wochen) im Leistungs-Zeitdiagramm oder aus der Spezifischen Trainingseffizienz nach einer der Gl. (11 bis 15, bzw. 16, 17) erkennbar. Dazu hat T in Gl. (11 bis 17) mindestens die Größe von einigen Wochen.

Zum Training werden auch stationäre Laufbänder und stationäre Trainingsgeräte verwendet, bei denen eine Messung der in der Trainingszeit t zurückgelegten Strecke s vorgenommen wird. Die gemessenen Parameter werden im erfindungsgemäßen Verfahren in Gleichungen (5, 6) verwendet.

Es werden auch stationäre Trainingsgeräte verwendet, bei denen eine Messung der im Training in der Zeit t umgesetzten Energie $E_B$ durchgeführt wird. Die gemessenen Parameter werden im erfindungsgemäßen Verfahren in Gleichung (6) verwendet.

Die praktische Durchführung wird nachfolgend in einer kurzen Darstellung tabellarisch beschrieben:

Die Herzleistung einer Person soll beim mehrfachen Zurücklegen einer vorgegebenen Strecke ermittelt werden. Dazu wird:

1. die Länge s der vorgegebenen Strecke gemessen,

2. zu einem Zeitpunkt $T_1$ eines erstmaligen Zurücklegens der Strecke s die Zeit $t_1$ für ein erstmaliges Zurücklegen der Strecke s gemessen,

3. die Körpermasse $m_1$ der Person vor dem erstmaligen Zurücklegen der Strecke s gemessen,

4. die Anzahl $n_{S1}$ der Herzschläge der Person während der Zeit $t_1$ des erstmaligen Zurücklegens der Strecke s gemessen,

5. zur Berechnung einer beim Zurücklegen der Strecke s erbrachten Bewegungsarbeit in Form mechanischer Arbeit $E_B$ als Produkt $E_B = m*a*s$ aus der Masse m der Person, der Beschleunigung a beim Zurücklegen der Strecke und der Länge s der Strecke, die Beschleunigung a als konstant (beispielsweise a = 1) gesetzt,

6. eine zum Zeitpunkt $T_1$ beim erstmaligen Zurücklegen der Strecke s erbrachte Bewegungsleistung in Form einer mechanischen Leistung $P_{B1}$ als Quotient aus der beim Zurücklegen der Strecke s erbrachten Arbeit $E_{B1} = m_1*a*s$ und der Zeit $t_1$ zu $P_{B1} = (m_1*a*s)/t_1$ ermittelt,

7. eine Herzleistung $P_{H1}$ zum Zeitpunkt $T_1$, als Quotient der beim erstmaligen Zurücklegen der Strecke s erbrachten Leistung $P_{B1}$ und der Anzahl $n_{S1}$ der in der Zeit $t_1$ erfolgten Herzschläge zu $P_{H1} = P_{B1}/t_1 = (m_1*a*s)/(t_1*n_{S1})$ ermittelt,

8. zu einem nachfolgenden Zeitpunkt $T_2$ bei einem nachfolgenden Zurücklegen derselben Strecke der Länge s die Zeit $t_2$ für das Zurücklegen der Strecke s gemessen, die Körpermasse $m_2$ der Person vor dem Zurücklegen der Strecke s gemessen und die Anzahl $n_{S2}$ der Herzschläge der Person während der Zeit $t_2$ des Zurücklegens der Strecke s gemessen,

9. eine zum Zeitpunkt $T_2$ beim nachfolgenden Zurücklegen der Strecke der Länge s erbrachte Bewegungsleistung in Form einer mechanischen Leistung $P_{B2}$ als Quotient aus der beim Zurücklegen der Strecke s erbrachten Arbeit $E_{B2} = m_2*a*s$ und der Zeit $t_2$ zu $P_{B2} = (M_2*a*S)/t_2$ ermittelt,

10. eine Herzleistung $P_{H2}$ zum Zeitpunkt $T_2$ als Quotient der beim erstmaligen Zurücklegen der Strecke s erbrachten mechanischen Leistung $P_{B2}$ und der Anzahl $n_{S2}$ der in der Zeit $t_2$ erfolgten Herzschläge zu $P_{H2} = P_{B2}/t_2 = (m_2*a*s)/(t_2*n_{S2})$ ermittelt,

11. aus dem Vergleich der Herzleistung $P_{H1}$ zum Zeitpunkt $T_1$, und der Herzleistung $P_{H2}$ zum Zeitpunkt $T_2$ eine Veränderung der Herzleistung zwischen den Zeitpunkten $T_1$, und $T_2$ ermittelt.

Alle weiter oben beschriebenen Einflüsse ("Tagesform", etc.) erfordern ausgleichende Maßnahmen. Der Ausgleich geschieht, indem nicht nur zu zwei Zeitpunkten $T_1$ und $T_2$ trainiert wird (die vorgegebene Strecke zurückgelegt wird), sondern sehr viel häufiger, d. h. regelmäßig in kurzen Abständen von ein bis zwei Tagen währen einer Trainingsperiode der Zeitdauer T von Tagen, Wochen oder Monaten wird dieselbe vorgegebene Strecke der Länge s zu einer Anzahl n von aufeinanderfolgenden Zeitpunkten $T_1, T_2, \ldots T_n$ wiederholt zurückgelegt. Zu jedem Zeitpunkt $T_1, T_2, \ldots T_n$ wird die Herzleistung $P_{H1}, P_{H2}, \ldots P_{Hn}$ beim Zurücklegen der Strecke der Länge s bestimmt. Aus dem Vergleich der Herzleistungen $P_{H1}$, $P_{H2}$,... $P_{Hn}$ wird die Entwicklung der Herzleistung während der Zeitpunkte $T_1$, $T_2$,...$T_n$ und während der Trainingsperiode T ermittelt.

Alternativ wird für eine Trainingsperiode T aus den Herzleistungen $P_{H1}$, $P_{H2}$,... $P_{Hn}$ für die Zeitpunkte $T_1$, $T_2$, ...$T_n$ eine zeitliche Änderung der Herzleistung $P_H$ innerhalb der Trainingsperiode der Zeit T als eine Herztrainingseffizienz $\eta_H$ in Form der ersten Ableitung der Herzleistung $P_H$, also $P_{H1}$, $P_{H2}$,... $P_{Hn}$ nach der Zeit T bestimmt.

Erfindungsgemäß wird eine Vorrichtung geschaffen, die die für das Verfahren notwendigen Parameter misst und speichert, nämlich den beim Üben zurückgelegten Weg s, die dafür benötigte Zeit t und die Zahl $n_s$ der dabei erfolgten Herzschläge. Ferner wird auch das Datum, (Tag, Monat, Jahr) aufgezeichnet. Für die erweiterte Ausführungsform werden auch die Zahl der Atemzüge $n_A$ und die Zahl der Schritte $n_{st}$ (bzw. weitere oder andere Bewegungsparameter) gemessen und aufgezeichnet. Hierzu werden technisch bekannte Vorrichtungen verwendet, wie anemometrische oder thermische Atemüberwachungssensoren, bzw. Schrittzähler, etc.. Außerdem wird zu jeder Übungseinheit die aktuelle Masse m des Übenden (mit Kleidung) bestimmt und der Vorrichtung eingegeben. Diese Vorrichtung wird in leicht tragbarer Bauweise ausgeführt und vom Übenden beim Training mitgeführt. Die Sensoren werden in technisch bekannter Weise am Übenden angebracht und verwendet. Erfindungsgemäß wird eine Personenwaage geschaffen, die das Gewicht beim Messvorgang automatisch an die Vorrichtung überträgt, wozu Waage und Vorrichtung über eine entsprechende, vorzugsweise drahtlose Kommunikationseinrichtung verfügen. Weg, Zeit und Zahl der Herzschläge, Atemzüge und Schritte, etc. werden parallel und zeitgleich gemessen, sodass Weg und die Zahl der Herzschläge (Puls), der Atemzüge und der Schritte, etc. als Funktion der Zeit t synchron erfasst und gespeichert werden. Datenerfassung und Speicherung werden vorzugsweise digital vorgenommen. Dazu verfügt die Vorrichtung über Analog-Digitalumsetzer (falls analoge Messwertgeber verwendet werden), digitale Speicher, sowie einen elektronischen Datenprozessor zur Steuerung der Messwerterfassung, Dateneingabe, Datenverarbeitung und Datenausgabe. Die Datenausgabe kann entweder auf Speichern erfolgen, und/oder auf einem zur Vorrichtung gehörendem Display, und/oder über drahtlose Datenübertragung auf ein Empfangsgerät.

Zur Wegmessung wird vorzugsweise ein Satelliten gestütztes Navigationssystem (z. B. Galileo, GPS, GLONASS) verwendet. Zur Messung der Zahl der Herzschläge wird vorzugsweise eine Vorrichtung nach Art der Pulsuhren eingesetzt, die gleichzeitig über eine Einrichtung zur Messung der Zeit und über einen Datenprozessor verfügt, der die Rechenoperationen nach Gln. (5 bis 17) ausführt. Zur Pulsuhr gehört auch der technisch bekannte Pulssensor (Pulsdatengeber), der an geeigneter Stelle am Körper getragen wird. Zur Messung der Zahl der Schritte wird vorzugsweise eine Vorrichtung nach Art der Schrittzähler oder der Laufsensoren eingesetzt, die alternativ zur Pulsuhr über eine Einrichtung zur Messung der Zeit und über einen Datenprozessor verfügt, der die Rechenoperationen nach Gln. (5 bis 17) ausführt. Im Arbeitsspeicher des Datenprozessors liegen dazu die Funktionen Gln. (5 bis 17) als Software vor.

Anstelle des Satelliten gestützten Navigationssystems kann zur Entfernungsmessung auch ein Laufsensor verwendet werden, bzw. die Wegstrecke auf andere technisch bekannte Weise gemessen werden.

Ein Ausführungsbeispiel der Erfindung wird anhand der Zeichnung näher erläutert.

Es zeigt:

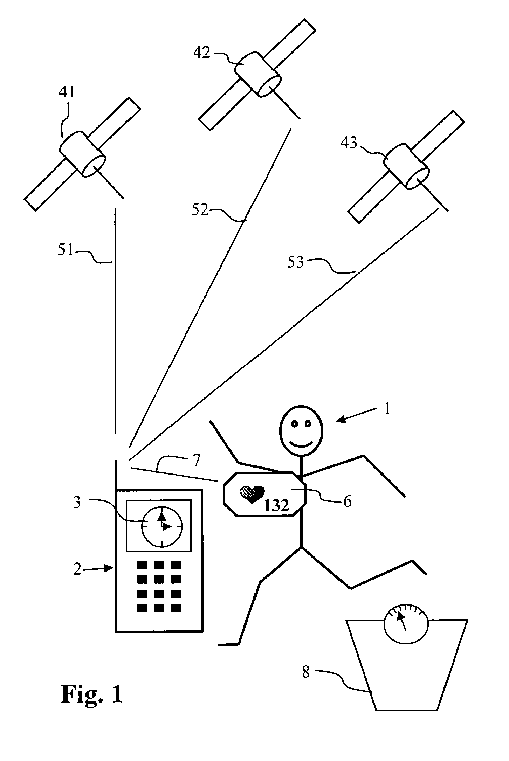

Fig. 1 eine schematische Darstellung einer Vorrichtung zur Bestimmung der Spezifischen Herzleistung $P_H$ und deren absoluten $\Delta P_H$ (T) und prozentualen Änderung $\Delta P_{Hp}$(T), sowie und der Spezifischen Herztrainingseffizienz $\eta_H$ aus der Spezifischen Gehleistung.

In der Zeichnung sind nur die wesentlichen Elemente der Vorrichtung dargestellt, bewusst ist auf die Wiedergabe von Einheiten zur Messwerterfassung, Dateneingabe, Speicherung, Datenverarbeitung und Datenausgabe, sowie von Bedienelementen verzichtet worden. Im Zentrum der Abbildung befindet sich, dargestellt als Strichmännchen, die trainierende Person 1 in schnellem Gehen. Sie führt ein System zur Messwerterfassung, Dateneingabe, Speicherung, Datenverarbeitung und Datenausgabe in Form eines Mobiltelefons 2 mit sich. Das Mobiltelefon **2** enthält alle Einheiten zur Messwerterfassung, Dateneingabe, Speicherung, Datenverarbeitung und Datenausgabe, die in der Abbildung aber nicht dargestellt sind. Das Mobiltelefon verfügt über eine Uhr **3,** symbolisiert durch ein Ziffernblatt im schematisch dargestellten Display des Mobiltelefons. Nicht dargestellt ist ein Kalender, der ebenfalls im Mobiltelefon enthalten ist. Ferner ist ein Satellitennavigationsempfangsgerät Bestandteil des Mobiltelefons, welches in der Abbildung nicht dargestellt ist. Diese Funktion wird symbolisiert durch drei schematisch wiedergegebene Navigationssatelliten **41, 42, 43** und deren Funkverbindungen **51, 52, 53** zum Mobiltelefon **2**. Die Person **1** trägt ein Pulsmessgerät **6** am Körper, welches seine Messdaten über die Datenverbindung **7** zum Mobiltelefon **2** überträgt. Fig. 1 enthält ferner eine Personenwaage **8,** mit der das Gewicht (die Masse) der Person **1** bestimmt wird. In nicht dargestellter Weise wird das Gewicht zum Mobiltelefon **2** übertragen. In diesem Ausführungsbeispiel überträgt also nur das Pulsmessgerät **6** seine Messdaten direkt zum Mobiltelefon **2** und der Messwert der Waage **8** wird dem Mobiltelefon **2** übergeben. Das Mobiltelefon **2** liefert selbst die Zeit, das Datum und die Navigationsdaten, aus denen letzteren die zurückgelegte Wegstrecke (und falls gewünscht das Höhenprofil) ermittelt werden.

In einer anderen Ausführungsform werden ein Empfangsgerät eines Satelliten gestützten Navigationssystems und eine Pulsuhr integriert und als eine kleine tragbare Einheit ausgeführt, die auch über eine Uhr, einen Kalender, eine Eingabeeinheit für die Messdaten der Waage, ein Display und einen Datenprozessor und -speicher verfügt.

In einer weiteren Ausführungsform werden die gemessenen Daten laufend oder periodisch an ein Mobiltelefon übertragen, welches teilweise die Funktion der erfindungsgemäßen Vorrichtung übernimmt und vom Übenden mitgeführt wird. Die Datenübertragung geschieht über die technisch bekannten Schnittstellen (usb, Klinke, wlan, bluetooth, manuelle Eingabe, etc.). Vorzugsweise verfügt das Mobiltelefon über ein Navigationssystem, eine Uhr und einen Kalender, sowie einen Datenprozessor und -speicher. Zur Übertragung werden die verschiedenen Messdaten in einer zentralen Messdatenverwaltungseinheit gesammelt und gemeinsam zum Mobiltelefon übertragen. Dazu verfügt die zentrale Messdatenverwaltungseinheit über eine zum Mobiltelefon kompatible Schnittstelle. Jede technisch bekannte Art der Datenübertragung kann verwendet werden.

Eine andere Ausführungsart hat die Form eines Datennetzwerkes, welches die genannten und auch andere Daten über den Körperzustand es Nutzers erfasst und verarbeitet (z.B. Körpertemperatur, Augenlidaktivität als Hinweis auf Müdigkeit eines Autofahrers). Im Speicher des Mobiltelefons werden die Daten abgelegt, in seinem Datenprozessor die Rechenoperationen nach Gln. (5 bis 17) ausgeführt. Im Arbeitsspeicher des Datenprozessors liegen dazu die Funktionen Gln. (5 bis 17) als Software vor. Die Ergebnisse werden im Speicher des Mobiltelefons gespeichert und auf dem Display bei Bedarf zur Anzeige gebracht. Anstelle des Mobiltelefons kann jede andere Art von Datenempfangs- und -verarbeitungssystemen verwendet werden (Pocket PC, Laptop, etc.).

In einem einfachen Anwendungsfall kann die Vorrichtung ohne Wegmesseinrichtung betrieben werden, wenn nämlich die Länge (und, falls gewünscht, das Höhenprofil) der Wegstrecke der Spezifischen Trainingseinheiten bekannt ist (z. B. Rundenlänge eines Sportplatzes, jede andere Strecke nach einmaliger Messung).

Im einfachsten Fall kann ganz auf eine Wegmessung verzichtet werden, nämlich wenn immer auf derselben Strecke trainiert wird und nur die Änderung der Herzleistung bestimmt werden soll.

In einer anderen Ausführungsform werden bekannte Beschleunigungsgrößen zur Berechnung der Energie nach Gleichungen (1) und (2) verwendet. Werden beispielsweise beim Gehen Steigungen und Gefälle überwunden, so werden diese bei der Berechnung der Energie durch Zerlegung in Komponenten berücksichtigt. Es wird der Anteil in der Ebene ergänzt durch den der Steigung und den des Gefälles:

$$E_B = m * a * s + m * g * h - m * b * h_G \qquad (18)$$

mit:

$h_S$: überwundene Höhe bei Steigung,
$h_G$: Höhendifferenz bei Gefälle,
b: Beschleunigung auf Gefällstrecke,
wobei a< g und g > b < a.

Beim Steigen einer Treppe der Höhe h mit reiner Hubarbeit reduziert sich die Gleichung (18) auf $E_B = m*g*h$. Wiederholtes Treppensteigen einer Treppe der Höhe h wird als Training verwendet. Die Gravitationsbeschleunigung g ersetzt dann den Parameter "const" in Gleichung (7).

Das Verfahren lässt sich auch bei Gartenarbeit wie dem Rasenmähen o. ä. anwenden. Dabei wird z. B. die mähspezifische Herzleistung bestimmt, in Quadratmeter je Sekunde und Herzschlag.

Als Erweiterung der Erfindung wird eine Spezifische Referenztrainingseinheit für jede Sportart definiert. Beim Gehen ist das eine Strecke bestimmter Länge, Steigung und Bodenbeschaffenheit, beispielsweise eine oder mehrere Runden eines Sportplatzes. Auf dieser Strecke werden durch breit angelegte reproduzierbare Messserien unter Laborbedingungen Referenzwerte der Spezifischen Herzleistung für verschiedene Alters-, Gewichts- und Leistungsgruppen ermittelt. Daraus werden Richtwerte für Trainingsziele für unterschiedliche Personen- und Altersgruppen (Männer, Frauen, Jugendliche, Sportler, Herzpatienten, etc.) abgeleitet, ähnlich der Gewichtsempfehlung in Abhängigkeit von der Körpergröße. Diese Richtwerte ergänzen die Trainingsempfehlungen der DGSP. Im Zusammenhang mit dem Messverfahren werden die Richtwerte beim Sport, sowie bei Rehabilitation und Prävention als Ziel und Kontrolle verwendet. Analog lassen sich Referenzdaten für auch für das Training an Geräten ermitteln und verwenden. Insbesondere wird auch Treppensteigen als Referenztrainingseinheit verwendet.

Über Vergleichsmessungen mit der Volumenförderleistung des Herzen und deren Veränderung im Training werden außerdem quantitative Bezüge zwischen beiden Größen hergestellt.

**Patentansprüche**

1. Verfahren zur Bestimmung einer Herzleistung einer Person beim mehrfachen Zurücklegen einer vorgegebenen Strecke, wobei

   - die Länge s der vorgegebenen Strecke gemessen wird,
   - zu einem Zeitpunkt $T_1$ eines erstmaligen Zurücklegens der Strecke s die Zeit $t_1$ für ein erstmaliges Zurücklegen der Strecke s gemessen wird,
   - die Körpermasse $m_1$ der Person vor dem erstmaligen Zurücklegen der Strecke s gemessen wird,
   - die Anzahl $n_{S1}$ der Herzschläge der Person während der Zeit $t_1$ des erstmaligen Zurücklegens der Strecke s gemessen wird,
   - zur Berechnung einer beim Zurücklegen der Strecke s erbrachten Bewegungsarbeit in Form mechanischer Arbeit $E_B$ als Produkt $E_B=m_*a_*s$ aus der Masse m der Person, der Beschleunigung a beim Zurücklegen der Strecke und der Länge s der Strecke, die Beschleunigung a als konstant gesetzt wird,
   - eine zum Zeitpunkt $T_1$ beim erstmaligen Zurücklegen der Strecke s erbrachte Bewegungsleistung in Form einer mechanischen Leistung $P_{B1}$ als Quotient aus der beim Zurücklegen der Strecke s erbrachten Arbeit $E_{B1}= m_{1*}a_*s$ und der Zeit $t_1$ zu $P_{B1}=(m_{1*}a_*s)/t_1$ ermittelt wird,
   - eine Herzleistung $P_{H1}$ zum Zeitpunkt $T_1$, als Quotient der beim erstmaligen Zurücklegen der Strecke s erbrachten Leistung $P_{B1}$ und der Anzahl $n_{S1}$ der in der Zeit $t_1$ erfolgten Herzschläge zu $P_{H1}=P_{B1}/t_1= (m_{1*}a_*s)/(t_1*n_{S1})$ ermittelt wird,
   - zu einem nachfolgenden Zeitpunkt $T_2$ bei einem nachfolgenden Zurücklegen derselben Strecke der Länge s die Zeit $t_2$ für das Zurücklegen der Strecke s gemessen wird, die Körpermasse $m_2$ der Person vor dem Zurücklegen der Strecke s gemessen wird und die Anzahl $n_{s2}$ der Herzschläge der Person während der Zeit $t_2$ des Zurücklegens der Strecke s gemessen wird,
   - eine zum Zeitpunkt $T_2$ beim nachfolgenden Zurücklegen der Strecke der Länge s erbrachte Bewegungsleistung in Form einer mechanischen Leistung $P_{B2}$ als Quotient aus der beim Zurücklegen der Strecke s erbrachten Arbeit $E_{B2}= m_2*a*s$ und der Zeit $t_2$ zu $P_{B2}=(m_2*a*s)/t_2$ ermittelt wird,
   - eine Herzleistung $P_{H2}$ zum Zeitpunkt $T_2$ als Quotient der beim erstmaligen Zurücklegen der Strecke s erbrachten mechanischen Leistung $P_{B2}$ und der Anzahl $n_{S2}$ der in der Zeit $t_2$ erfolgten Herzschläge zu $P_{H2}=P_{B2}/t_2= (m_2*a*s)/(t_2*n_{S2})$ ermittelt wird,
   - aus dem Vergleich der Herzleistung $P_{H1}$ zum Zeitpunkt $T_1$, und der Herzleistung $P_{H2}$ zum Zeitpunkt $T_2$ eine Änderung der Herzleistung zwischen den Zeitpunkten $T_1$, und $T_2$ ermittelt wird.

2. Verfahren nach Anspruch 1 wobei

   - während einer Trainingsperiode T von Tagen, Wochen oder Monaten dieselbe vorgegebene Strecke der Länge s zu einer Anzahl n von in Abständen von ein oder zwei Tagen aufeinanderfolgenden Zeitpunkten $T_1$, $T_2$, ...$T_n$ wiederholt zurückgelegt wird,
   - zu jedem Zeitpunkt $T_1$, $T_2$, ...$T_n$ die Herzleistung $P_{H1}$, $P_{H2}$, ...$P_{Hn}$ beim Zurücklegen der Strecke der Länge s bestimmt wird,
   - aus dem Vergleich der Herzleistungen $P_{H1}$, $P_{H2}$, ...$P_{Hn}$ die Entwicklung der Herzleistung während der Zeitpunkte $T_1$, $T_2$, ...$T_n$ in der Trainingsperiode T ermittelt wird.

3. Verfahren nach den Ansprüchen 1, und 2, wobei

   - für eine Trainingsperiode T aus den Herzleistungen $P_{H1}$, $P_{H2}$, ...$P_{Hn}$ für die Zeitpunkte $T_1$, $T_2$, ...$T_n$ eine zeitliche Änderung der Herzleistung $P_H$ innerhalb der Trainingsperiode der Zeit T als eine Herztrainingseffizienz $\eta_H$ in Form der ersten Ableitung der Herzleistung $P_H$, also $P_{H1}$, $P_{H2}$, ... $P_{Hn}$ nach der Zeit T bestimmt wird.

4. Verfahren nach den Ansprüchen 1, 2 und 3, wobei

   - das Zurücklegen der Strecke der Länge s als Spezifische Trainingseinheit in Form einer spezifischen Bewegungsart, wie Gehen, Laufen, Schwimmen, Radfahren, Skilanglauf, Rudern, Rollerskating, Skateboarding,... erfolgt.

5. Verfahren nach den Ansprüchen 1, 2, 3 und 4 wobei

   - bei jeder Spezifischen Trainingseinheit eine Spezifische Lungenleistung $P_L$ ermittelt wird als Quotient aus der Bewegungsleistung $P_B$ und der in der benötigten Zeit t erfolgten Zahl der Atemzüge $n_a$ und

- dass zu jeder Spezifischen Trainingseinheit zeitliche Änderungen der Spezifischen Lungenleistung $P_L$ innerhalb der Trainingsperiode der Zeit T als eine Spezifische Lungentrainingseffizienz $\eta_L$ in Form der ersten Ableitung der Spezifischen Lungenleistung $P_L$ nach der Zeit T bestimmt werden;

- dass zu jeder Spezifischen Trainingseinheit der Art Gehen oder Laufen eine Spezifische Schrittleistung $P_s$ ermittelt wird als Quotient aus der Bewegungsleistung $P_B$ und der in der benötigten Zeit t erfolgten Zahl der Schritte $n_{st}$ und

- dass zu jeder Spezifischen Trainingseinheit zeitliche Änderungen der Spezifischen Schrittleistung $P_s$ innerhalb der Trainingsperiode der Zeit T als eine Spezifische Schritttrainingseffizienz $\eta_S$ in Form der ersten Ableitung der Spezifischen Schrittleistung $P_s$ nach der Zeit T bestimmt werden.

6.  Verfahren nach den Ansprüchen 1, 2, 3, 4, und 5, wobei,

- zu jeder Spezifischen Trainingseinheit eine Änderung der Spezifischen Leistung, in Form einer Herzleistung, spezifischen Lungenleistung, spezifischen Schrittleistung innerhalb der Trainingsperiode der Zeit T durch Bezug auf einen Referenzwert quantitativ ermittelt wird,

- dass der Referenzwert der Mittelwert der Spezifischen Leistung in der Spezifischen Trainingseinheit innerhalb der Trainingsperiode der Zeit T ist,

- dass alternativ der Referenzwert der Anfangswert der Spezifischen Leistung in der Spezifischen Trainingseinheit innerhalb der Trainingsperiode der Zeit T ist.

7.  Vorrichtung zur Durchführung des Verfahren nach den Ansprüchen 1 bis 6, **dadurch gekennzeichnet,**

- **dass** ein Pulsmessgerät 6, ein Wegmessgerät und eine Uhr 3 mit Kalender zur Übertragung, Speicherung und Verarbeitung ihrer Messwerte mit einer Datenerfassungs- und -verarbeitungseinrichtung gekoppelt sind,

- **dass** die Einrichtung über eine Eingabeeinheit zur Eingabe weiterer Messwerte, wie die Masse eines Übenden, die von einer Waage 8 gemessen wird, verfügt,

- **dass** die weiteren Messwerte gespeichert und verarbeitet werden können,

- **dass** die Datenverarbeitungseinrichtung aus den Messwerten die Herzleistung als Funktion der Zeit und die Spezifische Trainingseffizienz berechnet.

8.  Vorrichtung nach Anspruch 7, **dadurch gekennzeichnet,**
    **dass** das Wegmessgerät ein Satelliten gestütztes Navigationssystem **41, 42, 43,** ist, und
    **dass** das Wegmessgerät alternativ ein Laufsensor ist.

9.  Vorrichtung nach Anspruch 7, **dadurch gekennzeichnet,**
    **dass** die Datenerfassungs- und -verarbeitungseinrichtung ein Mobiltelefon 2 ist.

10. Vorrichtung nach Anspruch 7, **dadurch gekennzeichnet,**
    **dass** ein Schrittzähler und ein Atemüberwachungssensor mit der Datenerfassungs- und

    - verarbeitungseinrichtung gekoppelt sind.

11. Vorrichtung zur Durchführung des Verfahrens nach den Ansprüchen 1 bis 7, **dadurch gekennzeichnet,**

- **dass** die Vorrichtung ein stationäres Trainingsgerät mit integrierter Messung der in der Trainingszeit t zurückgelegten Strecke s ist.

- **dass** die Vorrichtung ein stationäres Trainingsgerät mit integrierter Messung der in der Trainingszeit t umgestzten Bewegungsenergie $E_B$ ist.

12. Verfahren zur Bestimmung der Herzleistung einer Person beim mehrfachen Zurücklegen einer Strecke nach Anspruch 1 wobei,

- bei der Ermittlung der erbrachten Bewegungsarbeit in Form mechanischer Arbeit $E_B$ bekannte Beschleunigungswerte a verwendet werden und

- dazu als zurückgelegte Strecke eine Treppe der Höhe h verwendet wird, wobei als Beschleunigung die Gravitationsbeschleunigung g wirkt und die mechanische Arbeit als Produkt $E_B = m * g * h$ ermittelt wird.

**13.** Verfahren zur Bestimmung der Herzleistung von Personen nach Anspruch 1, **dadurch gekennzeichnet,**

- **dass** mittels Spezifischer Referenztrainingseinheiten, in Form reproduzierbarer Trainingsabläufe und -strecken Referenzwerte für die Herzleistung von Personen unterschiedlichen Alters und Geschlechts ermittelt werden, und
- **dass** aus den Referenzwerten Richtwerte für Trainingsziele abgeleitet werden und
- **dass** Treppensteigen als Referenztrainingseinheit verwendet wird.

**Fig. 1**